# EUROPEAN PATENT SPECIFICATION

(11) **EP 2 630 980 B1**
(45) Date of publication and mention of the grant of the patent: **29.08.2018**
(21) Application number: 13156477.5
(22) Date of filing: 19.03.2008
(51) Int. Cl.: A61M 5/20, A61M 5/172, A61B 18/08, A61M 5/158, A61M 5/142, A61M 5/30, A61M 5/42, A61F 7/00, A61B 18/14, A61B 18/18, A61B 18/20, A61N 1/30, A61M 5/168, A61N 7/00, A61N 5/06

(54) **Drug delivery device**
Arzneimittelabgabevorrichtung
Dispositif d'administration de médicaments

(30) Priority: 19.03.2007 US 895518 P; 19.03.2007 US 895519 P; 19.04.2007 US 912698 P; 30.05.2007 US 940721 P; 21.06.2007 US 821230; 18.12.2007 US 8277; 25.12.2007 US 16571; 10.01.2008 US 10758
(43) Date of publication of application: 28.08.2013
(62) Divisional of application: 08719774.5
(73) Proprietor: Insuline Medical Ltd., 49002 Petach-tikva (IL)
(72) Inventor: Pesach, Benny, 48072 Rosh-ha Ayin (IL); Bitton, Gabriel, 97279 Jerusalem (IL); Nagar, Ron, Tel Aviv, 62198 (IL); Weiss, Ram, 34485 Haifa (IL)
(74) Representative: Cooley (UK) LLP

(56) References cited:
- EP-A1- 1 752 174
- WO-A-00/32259
- WO-A1-00/78212
- WO-A2-2005/016401
- WO-A2-2006/049570
- WO-A2-2006/091650
- US-A1- 2004 215 173
- US-A1- 2005 256 499

## Description

### CROSS-REFERENCE TO RELATED APPLICATION

### BACKGROUND OF THE INVENTION

### Field of the Invention

The present invention relates to systems for delivering drugs to a patient. In particular, the present invention relates to systems for subcutaneous injection of a medicament and using one or more treatment sources to improve effectiveness of the injected drugs.

### Background of the Invention

Pen injectors are useful when regular injection by persons without formal medical training occurs. This is increasingly common amongst those having chronic conditions such as diabetes where self-treatment enables such persons effectively manage their condition. Many of the insulin pen injectors are reusable and usually loaded with an insulin cartridge that may be used for a plurality of injections or for a number of days. Many other diabetic patients use regular syringe(s) and needles for insulin injection. An example of a device for intramuscular or subcutaneous injections is described in WO 00/32259.

Diabetes is a very serious illness affecting millions of people today. Many diabetic patients require injections of insulin to maintain proper levels of glucose in their blood in order to survive. Such injections of insulin require drug injection systems.

Many medical treatment systems and methods involve drug injection systems that employ subcutaneous injections of therapeutic fluids, drugs, proteins, and other compounds. Such delivery systems and methods, especially for insulin delivery, may use injection pens to inject insulin to the subcutaneous tissue, or regular syringe. In the conventional insulin injection pens, the pen includes a disposable insulin reservoir and a disposable needle through which insulin is injected into the tissue. The needle is a single use needle, while the insulin reservoir can be used for two to three days. In the conventional insulin injection pens, the injection is done by attaching the insulin injection pen to the skin at the injection site and pressing a button that first insert the needle using a spring into the subcutaneous tissue and then inject the insulin to the subcutaneous tissue.

In many instances, the patients require insulin injection around the clock to keep proper levels of glucose in their blood. Two major types of insulin can be injected - a long-acting insulin that provides the basal insulin rate needed for keeping patient's blood glucose in the desired range between meals and over night and an insulin bolus injection that provides an amount of insulin for matching a dose of carbohydrates consumed by the patient.

When patient consumes food, his or her levels of glucose rise. Unfortunately, many conventional subcutaneous injection devices are incapable of quickly matching and/or preventing the rise of blood glucose. The delay in such matching is also true in case of the "rapid-acting" insulin. Some of the reasons for this delay include a lag in the absorption of insulin from the injection site and the time it takes for complex insulin molecules to break down into monomers.

Additionally, since blood glucose levels rise shortly following the meal, the delay in matching insulin to the rising levels causes post prandial hyperglycemic events (i.e., when levels of blood glucose are above normal) to occur. Occasionally, after certain period of time passes (e.g., 2-3 hours) after the meal, the blood glucose levels drop yet insulin concentrations in the blood rise followed by the peak of the systemic insulin effect and may result in causing hypoglycemic events (i.e., when levels of blood glucose are below normal) to occur. Both hyperglycemic and hypoglycemic events are highly undesirable. Additionally, since local blood perfusion at the insulin injection region has large variability, depending on the ambient temperature and other parameters, it induces large variations to the delay of the peak of time profile of the insulin action. Those variations in the insulin peak action period further increase the variability in the blood glucose level.

Thus, it is desirable to provide a system and a method that provides efficient and rapid injection and absorption of the drug to the patient circulatory system. In particular, it is desirable to provide a system and a method for injection of insulin to the patient that improves effectiveness of insulin in the blood to maintain normal levels of blood glucose and prevent or reduce hyperglycemic and hypoglycemic events.

### SUMMARY OF THE INVENTION

The present invention relate to systems and devices for injecting a drug, substances and/or chemicals to a patient that further provides a tissue treatment element for improving the effectiveness of drug delivery upon injection. A method for injecting a drug is also disclosed. In some embodiments, the present invention relates to a device for improving performance of drug delivery in the form of injection pens or syringes. In general, the present invention's suggested devices can be used in many drug injection devices, such as injection pen(s), syringe(s), or jet injector(s), or other injection devices. As such, although the present application discusses mainly injection pens, it is understood by one skilled in the art that such devices can be used with any other injection devices. In some embodiments, the present invention provides for a device that further provides an additional treatment to a tissue region where the drug is delivered. In some embodiments, the treatment is utilized to improve drug delivery process by improving the drug's pharmacokinetic and/or pharmacodynamic profile. The treatment may come in various forms, for example, including analgesic, vasodilator or the like. The treatment may be any form of treatment that leads to improved vasodilatation of the tissue being injected, including but not limited to, exposing the tissue region to an energy, radiation, heat, mechanical vibrations, suction, massaging, acoustic stimulation, electromagnetic radiation, electric field, magnetic field, electrical stimulation, injection of an additional substance(s), or any combination of the above to improve the drug's pharmacokinetic and /or pharmacodynamic profile. Each treatment type may have a separate protocol in order to evoke the necessary reaction such as vasodilatation or the like.

In some embodiments, the present invention provides a needle free drug delivery pen that is coupled to a treatment element. The treatment element improves the pharmacokinetic and/or pharmacodynamic properties of the drug that is being delivered to the target tissue using a fluid jet. The drug delivery injector for administering a drug, for example, insulin, as a jet nozzle configured for firing insulin in a fluid jet in a configuration and with sufficient velocity to penetrate tissue of the patient to a delivery site. A drug containing compartment is associated with the nozzle for containing the drug and feeding the insulin to the delivery nozzle for injection. A firing mechanism includes an energy source is associated with the drug compartment for forcing the drug through the nozzle at a sufficient velocity to penetrate to the target site. In some embodiments, the energy source producing the fluid jet can be a coil spring, gas spring, or any other spring. A trigger or a dosage release button of the drug delivery injector is movable by the user and associated with the firing mechanism for activating the energy source that produces the drug fluid jet by forcing of the drug through the nozzle once the release button is activated.

In some implementations, the applied treatment induces vasodilatation through neural stimulation of the tissue of the drug injection site. The neural stimulation can be induced by thermal stimulation and/or mechanical stimulation and/or chemical stimulation. The human neural response to the thermal stimulation includes several mechanisms such as the Nociceptive Axon Reflex that induce vasodilatation among other effects.

In some implementations, the induced neural response, such as the Nociceptive Axon Reflex, also induces widening of the capillary pores and increasing the capillary wall permeability. This effect is also significant for improving the absorption of the drag through the capillary wall.

In some implementations, the applied treatment may lead to a reduction in the variability of the drug absorption in the blood or lymph system and its local and systemic effects. For example, heating the tissue region in the vicinity of the area of drug delivery to a preset regulated temperature during and/or after the drug injection and absorption into the blood may cause local blood perfusion at that region to become more reproducible and the drug absorption process more uniform and reproducible as well. Also, by reducing the delay between drug injection into the tissue and absorption into the blood system, the variability of drug action induced by the delayed profile can be reduced. In some embodiments, the temperature of the region adjacent to the injection region can be regulated for longer periods, but the cost may be the energy source volume and weight. Therefore, for minimization of the energy source size, the heating period or heating temporal profile can be optimized in relation to the period of the drug injection and absorption into the blood. In some embodiments, in which the treatment utilized is, for example, heat, the drug interaction with the treatment substance or type will be taken into consideration and can be avoided. For example, a drug's temperature sensitivity will be accounted for so as to avoid protein denaturisation. Insulin is a temperature-sensitive protein and to avoid damage to the insulin the treatment protocol, heating can be limited to ensure the efficacy of the delivered drug. For example, the treatment protocol may control the temperature or the location of the treatment delivery site so as to not damage the drug.

In some implementations, the neural response that induces vasodilatation is stimulated by applying a mechanical force in the vicinity of the drug infused region, wherein the force includes, but is not limited to, one or more of the following: pressure, massage, vibration, suction and/or any other mechanical stimulation. These tissue treatments or stimulations are known to stimulate the Nociceptive Axon Reflex as well. Among the advantages of the mechanical stimulation is the fact that it does not damage the drug, whereas for example heating insulin above 37°C may cause damage to it. The calibration of the applied mechanical force may be performed by using one of the procedures discussed above.

In some implementations, an additional fluid substance can be combined with the drug or, alternatively, injected, infused, or topically applied (which may include transdermal delivery of the drug by permeating through the skin of the patient) to the drug injection site, such that the additional substance induces neural stimulation that leads to local vasodilatation and/or increases of the capillary permeability. The substances can include tolazine, naftidrofuryl, suloctidil, nitroprusside, capsaicin, or any other suitable substance. In some implementations, an additional substance may induce vasodilatation and improve blood perfusion in the drug infused tissue region. For example, capsaicin stimulates a neural response through the VR1 receptor and produces a similar response to thermal neural stimulation.

The treatment element can be an integral part of the drug delivery injection pen, according to some embodiments of the present invention. In some embodiments, the treatment element can be an auxiliary unit that may be interchanged, replaced, or added to an existing drug delivery injection pen. Such a device can be attached to the drug delivery pen either during or before the drug injection or applied to the drug injection site afterward.

The treatment element, according to some embodiments, may be any one or more of (or a combination of): a heating element, a radiation emitter, a sound transducer, a mechanical/electo-mechanical vibration device, a light emitting device, and an electrode.

In some embodiments, one or more of properties relating to the treatment element may be controlled by a processor in order to achieve a desired response of the tissue region undergoing drug delivery. Such properties include amplitude, phase, frequency, combination of excitation sources, relative ratio and timing between various excitation sources, or any other properties. In some embodiments, the treatment type or sources can be also adjusted according to chemical and/or physical properties of the drug being delivered. The tissue response to the treatment element/stimulation enhances the functionality of the injected drug by enhancing the kinetics of molecular transport from the injection site inside the tissue to various compartments surrounding the tissue region and to the blood system.

In some embodiments, a treatment element or device supplying tissue treatment or stimulation to a tissue region can be configured to monitor and control properties of the treatment source. For example, controllable properties of a treatment protocol include amplitude, phase, intensity, frequency, or any other properties. Further control can be gained by actively monitoring, such that the information is provided to a controller ("controller" or "processing unit") that uses the information to reduce the variability of the drug pharmacokinetics. In such embodiments, the device can be configured to monitor properties of the adjacent tissue, such as local blood perfusion or skin temperature. Based on such monitoring, the information can be provided to the controller that utilizes the information to improve pharmacokinetic or pharmacodynamic profile of the drug as well as its performance and reduce variability of the drug injection process.

In some embodiments, the present invention's device includes a sensor or other triggering input mechanism that is configured to prevent deployment of the drug delivery pen unless certain criteria are fulfilled. Such criteria can include activation of a treatment protocol or element.

In some implementations, tissue treatment can be applied simultaneously with each injection of the drug delivery. In other embodiments, the tissue treatment or stimulation option may be selected manually by the user. In some embodiments, the user may choose to attach the treatment element to the drug delivery pen. The user can enable or disable mechanically the automatic application of treatment element. The user can activate the treatment device or devices before or after the drug injection to enhance the tissue response to the injected drug. Such activation can be done by pressing a button or a sequence of buttons on the drug delivery pen.

For example, in case of an insulin delivery pen, the pen may have a special button for triggering a "fast bolus" as compared to regular bolus injection provided by the drug delivery injection pen. The fast insulin bolus mode can be configured to start one of the above treatments parallel to the injection of insulin or short time before or after the injection of the insulin bolus for a given period of time. This improves or modifies pharmacokinetics or pharmacodynamics of insulin administration, tissue blood perfusion and/or absorption in the blood of a patient and is highly advantageous when applied in conjunction with high glycemic index foods. Application of a "fast bolus" may be useful for consumption of high glycemic index foods, where larger rapid glucose excursions occurs, but also in most of the cases of using insulin boluses for prandial coverage. In some embodiments, application of a "fast bolus" can be set as the default mode of the drug delivery pen. In some implementations, the user may apply the tissue treatment or stimulation before the meal to further increase the treatment effect.

In some implementations, at least one effect of the treatments is to reduce local irritation caused by the infused drug or local inflammation reaction caused by the injection. For example, in case of insulin injection, reducing the period in which the high concentration of insulin remains in the tissue may reduce irritation that may be caused by insulin. It can also reduce unwanted effects of the insulin delivery, such as, lipohypertrophy.

Some implementations also provide methods for improving or modifying a drug's pharmacokinetic or pharmacodynamic profile in order to reduce time to peak action in the blood of the injected material by applying a modulation pattern to the infused drug. With this modulation, the injection drug fluid is slightly moved/pulled in and out of the tissue during or after the drug injection process. In such implementations, this method may not require an additional device applied to the skin.

In some embodiments, the drug delivery pen can mechanically attach a small disposable device to the skin either before, during or after delivery of the drug. The disposable device can apply a treatment or treatments using at least one of the following sources: a heat source (such as a heat resistor), a suction port, for example activated by a pump, a mechanical vibration source, an ultrasound excitation source, an ultrasound transducer, a light source, a massaging element, electromagnetic radiation source, electric field source, magnetic field source, additional substance and/or a combination of at least two of sources to improve drug pharmacokinetics. In some embodiments, the small disposable device can be attached manually either before or after injection of the drug.

In some embodiments, a device for drug injection includes a disposable injection needle for injecting drug into tissue, a reusable drug delivery pen for inserting the needle into the patient skin or subcutaneous layer and for injection of the drug through the needle into one of the skin and/or subcutaneous tissue layer, a treatment device for applying a specific treatment or stimulation to the drug injected region in order to improve drug's pharmacokinetic, pharmacodynamic profile and/or to increase blood perfusion in that region before, during and/or after the drug injection period to improve drug absorption into the blood system. The needle can be injected automatically at the target site using an automatic needle triggering piston or spring. In some embodiments, the needle can be injected at the target site manually through the action of the user inserting the needle independently.

In some embodiments, a device for drug injection includes an injection catheter for insertion into the tissue, a drug injection device for infusing a drug into the injection catheter, a treatment device for applying a specific treatment or stimulation to the drug infused region in order to improve, modify and/or stabilize the drug pharmacokinetics, pharmacodynamics, and/or to reduce variations of the drug absorption into the blood system.

In some embodiments, a device for drug injection includes at least one of the following: a display, a button, a memory for boluses, a processing unit, a sensor for skin properties, a sensor for treatment level, a glucose sensor, a user interface, wireless connection to a PDA or cell phone for having memory and reminders and remote access to support sites.

In some embodiments, the device for drug/insulin injection includes a glucose sensor. The glucose sensor may measure blood glucose level at alternate sites (for example, at sites with reduced blood perfusion, such as arms and legs). The glucose sensor can be provided on the opposite side the injection end.

In some embodiments, the present invention can be configured for a jet injection. Jet injection involves high pressure injection of material, which obviates the use of needles. This type of injection mode is also referred to as "needle free" or "needleless" injection. In some embodiments, the pen injection device can include a jet injection, in addition to or in place of, the use of one or more needles. Some examples of conventional needle-free injection systems include the Medi-Jector VISION® and some products by Antares. Such systems can be adapted for use with the present invention's jet injection system.

In some embodiments, the injection device includes a disposable nozzle and a reservoir having an additional substance. The reservoir is located at the nozzle and the additional substance is provided in a single use or single dose amount. The reservoir is located within the body of the device and the nozzle features a connector for fluid or other communication with the reservoir.

In some embodiments, rather than disposing a nozzle along with an additional reservoir, an applicator for an additional substance is provided that is attached to or separate from the device. The nozzle can be disposed along with a gauge for adjusting the amount of additional substance to be applied. The applicator may be controlled through a button or other control component. In some embodiments, the gauge can be configured as a ring that can be rotated around the applicator button or other control device to adjust the amount that the button is pressed and/or some function of the other control device and/or to adjust the dose of applied additional substance.

In some embodiments, the drug delivery pen can include an adhesive material, such as a sticker, for assisting the user to create a skin fold for administration of the drug and/or additional substance.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in order to provide what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.
Figures 1A-E illustrate an exemplary drug delivery pen combined with a mechanism for topical application of an additional substance to the drug injection site, according to some embodiments of the present invention.
Figures 2A-C illustrates an exemplary drug delivery pen combined with a mechanism for topical application of an additional substance to the drug injection site, according to some embodiments of the present invention.
Figures 3A-C illustrates an exemplary drug delivery pen combined with a mechanism for topical application of an additional substance to the drug injection site, according to some embodiments of the present invention.
Figures 4A-D illustrates an exemplary drug delivery pen combined with a mechanism for topical application of an additional substance to the drug injection site, according to some embodiments of the present invention.
Figures 5A-C illustrates an exemplary drug delivery pen combined with a mechanism for application of a treatment element on the drug injection site, according to some embodiments of the present invention.
Figures 6A-C illustrates an exemplary drug delivery pen combined with a mechanism for application of a treatment element on the drug injection site, according to some embodiments of the present invention.
Figures 7A-C illustrates an exemplary drug delivery pen and cover combined with a mechanism for application of a treatment element on the drug injection site, according to some embodiments of the present invention.
Figures 8A-D illustrates an exemplary drug delivery pen combined with a mechanism for application of a treatment element on the drug injection site, according to some embodiments of the present invention.
Figures 9A-C illustrates an exemplary drug delivery pen combined with a mechanism for application of a treatment element on the drug injection site, according to some embodiments of the present invention.
Figures 10A-F illustrates an exemplary treatment element that may be coupled to a drug injection at the drug injection site, according to some embodiments of the present invention.
Figures 11A-D are block diagrams of exemplary drug delivery devices, according to some embodiments of the present invention.
Figures 12A-C illustrate an exemplary drug delivery pen combined with a mechanism for application of a treatment element on the drug injection site, according to some embodiments of the present invention.
Figure 13 is a flow chart illustrating an exemplary method for controlling temperature of heating that is provided by a treatment element in order to prevent degradation of a temperature sensitive drug.

### DETAILED DESCRIPTION OF THE INVENTION

The present invention relates to a drug delivery pen or other drug injection devices for the injection of a drug at a drug injection site, where the drug in injection device applies a treatment that can improve injected drug's pharmacokinetic and/or pharmacodynamic properties. The following description will refer to a drug injection pen for illustrative, nonlimiting purposes, however, as can be understood by one skilled in the art, the present invention is applicable to any other drug injection devices.

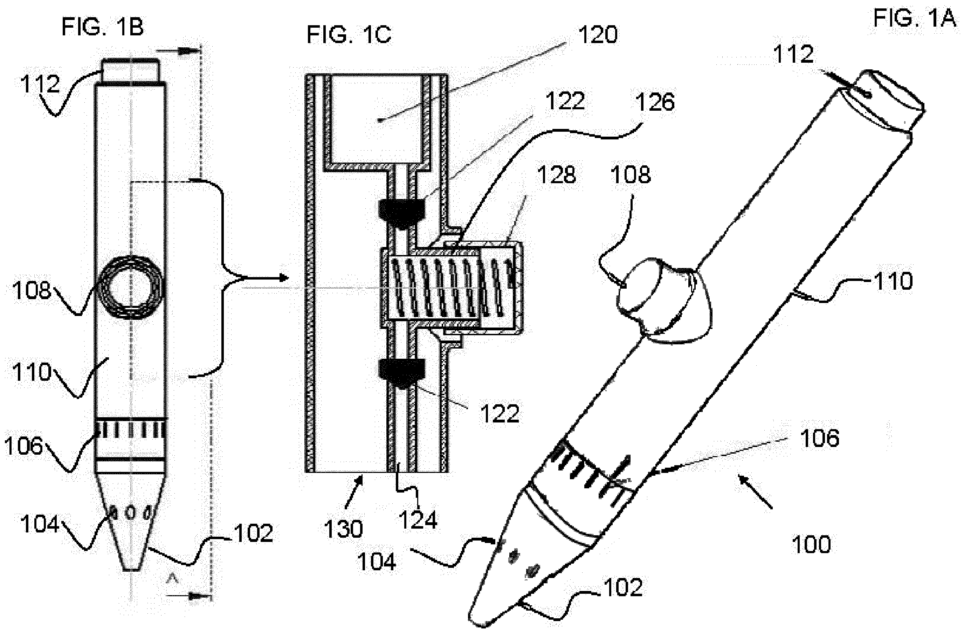

Figures 1A-C are schematic diagrams of an exemplary drug delivery pen 100 having a treatment element coupled to the delivery pen, according to some embodiments of the present invention. Figures 1A and 1B depict an exemplary drug delivery pen 100 having a pen shaft 110, an injection piston release trigger/button 112 (for actuating a piston pump, for example, within the housing of the delivery pen), a treatment release trigger/button 108 (for actuating a pump for delivering the treatment), a needle opening and a housing 102, treatment delivery openings 104, and a drug dosage selector 106. Figure 1A is a perspective view of the drug delivery pen 100. Figure 1B is a side view of the pen 100.

The drug delivery pen, for the delivery of insulin, may function as do state of the art drug delivery pens by injecting the selected and determined drug dosage 106 and using injection piston release button 112 to release the piston (not shown) that presses and/or otherwise places pressure on the drug reservoir syringe or vial, for causing injection of the required dosage of drug (not shown) into the targeted area and through needle housing 102. In some embodiments, a fluid jet of the delivered drug can be utilized instead of a syringe to delivery the drug through housing 102 to the targeted delivery site. The drug delivery pen can provide an additional treatment element, such as, an anesthetic, a drug for inducing treatment, a drug for improving effectiveness of the primary drug being injected. The primary drug can be insulin.

The treatment can be delivered over the target tissue using the treatment delivery openings 104, wherein a fluid treatment substance can be applied or sprayed onto the tissue. Figure 1C is a cross-sectional view of the drug delivery pen 100 showing the treatment element 130 and components thereof. Treatment element 130 includes treatment compartment 120, a plurality of valves 122, a pump 126 and a treatment release button 128. In some embodiments, the treatment compartment 120 stores a formulation of a liquid, ointment, solution, aerosol, foam, solid, gel foam, pressurized liquid, gas, spray, pain reliever drug, analgesic, vasodilatation drug, septic, alcohol, or the like for treatment of the skin tissue area. In some implementations, treatment may be applied prior to or following deployment of the needle.

Treatment substance stored in compartment 120 can be pumped to the treatment release openings 104 using schematic pump 126 that is activated by pressing treatment release button 128. The treatment release button 128 releases valves 122 to deliver treatment liquid from the compartment 120 through the tube 124 that leads to openings 104. The treatment applied to an area can lead to improved pharmacokinetic and/or pharmacodynamic properties of the primary drug being delivered by way of injection. The pathway by which the pharmacodynamic and/or pharmacokinetic properties of the drug are improved may be dependent on the treatment substance or element utilized.

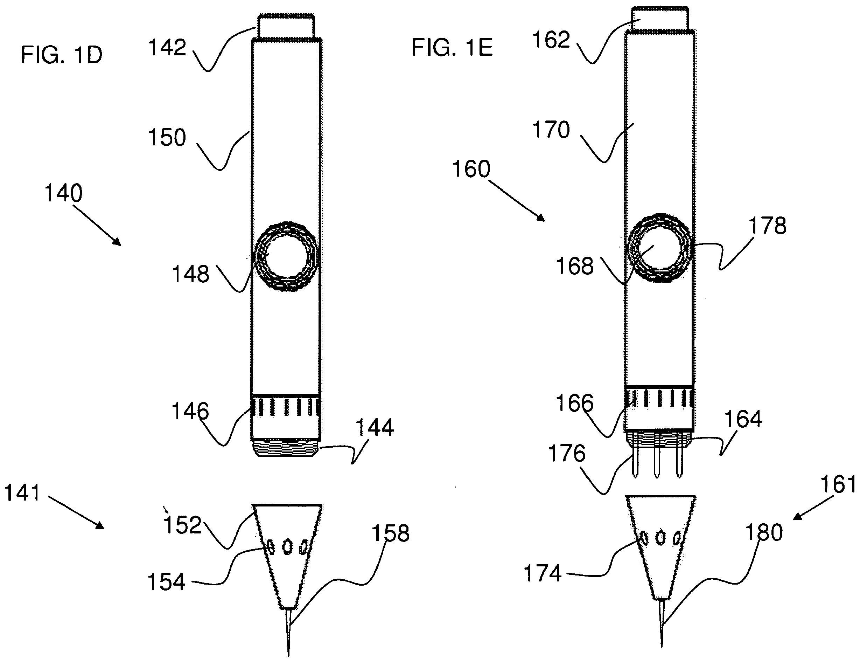

Figure 1D is a partial exploded view of a drug delivery pen 140 having a manual injection portion 141, according to some embodiments of the present invention. Pen 140 includes a pen body 150, a dosage release button 142, a treatment release button 148, a drug dosage selector 146, and an injection coupling portion 144. Injection coupling portion 144 is a connection mode to securely affix injection portion 141 to the pen portion 140. The coupling of the injection portion 141 to the pen portion 140 may be accomplished by at least one or more connecting pieces, such as, male/female connectors, threaded connectors, snap connector(s), turn key connectors, hook connectors or any other suitable connectors.

Manual injection portion 141 includes a treatment substance reservoir 152, a treatment delivery opening 154 and a needle 158. The manual injection portion 141 can be disposable after a single use. However, the injection portion 141 can be reusable and can be used a number of times or repeatedly over a period of time. Prior to drug delivery the drug delivery pen body 150 is securely coupled to manual injection portion 141 allowing a user to use the drug delivery pen 140 by selecting a drug dosage using dosage selector 146. Treatment release button 148 triggers the release of the treatment substance from the treatment reservoir 152 through delivery openings 154. The timing of treatment release can be performed prior to, during or following drug delivery, or in a combination thereof. Drug delivery can be accomplished by setting the dosage amount using dosage selector 146, manually inserting needle 158 into the target site, releasing treatment substance through delivery openings 154 and finally delivering the drug with dosage release button 142.

Figure 1E depicts an additional optional embodiment of the manual drug delivery pen of Figure 1D. Manual drug delivery pen includes a drug delivery pen housing 160 and manual injection portion 161. Pen housing 160 includes a pen body 170, a dosage release button 162, a drug dosage selector 166, a treatment release button 168, a treatment dosage selector 178, a treatment substance tube 176, and an injection coupling portion 164. The injection coupling portion 164 can be configured to securely affix injection portion 161 to the pen body portion 160. Coupling of the injection portion 161 to pen portion 160 can be accomplished by at least one or more connecting pieces, such as, a male to female connector(s), threaded connectors, snap connector(s), turn key connector(s), hook connectors or any other connection devices.

Manual injection portion 161 is disposable for single use, however, injection portion 161 may be used a number of times, or repeatedly over a continuous period of time. Prior to drug delivery the drug delivery pen body 170 is securely coupled to manual injection portion 161 allowing a user to use drug delivery pen 160 by selecting a drug dosage using drug dosage selector 166. Treatment dosage selector 178 determines the dosage of the treatment substance to be released with treatment release button 168. Pressing treatment release button 168 triggers the release of the treatment substance from the treatment reservoir (not shown) through at least one or more treatment delivery tube 176 that ends in treatment delivery openings 174. Timing of treatment dosage selection and release may be performed prior to, during or following drug delivery, or in a combination thereof. Drug delivery is accomplished by setting the dosage amount using dosage selector 166, manually inserting needle 158 into the target site, releasing treatment substance through delivery openings 174 as described above and finally delivering the drug with dosage release button 162.

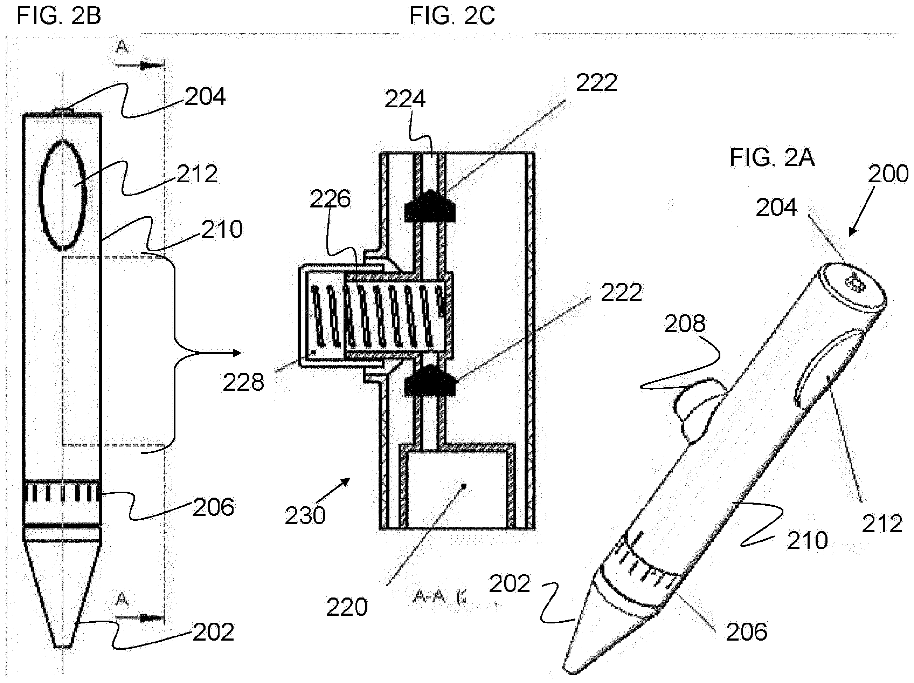

Figures 2A-C illustrate an exemplary drug delivery pen according to the present invention which is similar to the embodiments depicted in Figures 1A-C. In Figures 2A-C embodiments, the treatment opening is on the opposite side of the needle housing. Figures 2A and 2B depict a drug delivery pen 200 including a pen shaft 210, an injection release button 208, a treatment release button 212, a needle opening and a housing 202, a treatment delivery opening 204 and a drug dosage selector 206. Figure 2A is a perspective view of the drug delivery pen 200. Figure 2B is a side view of pen 200.

The drug delivery, such as insulin delivery, functions in a similar fashion as some conventional pens, such as NovoPen, FlexPen, Sanofi Aventis pens or the like, by setting the determined drug dosage 206 and pressing an upper button (not shown in Figures 2A-C) that pushes the syringe piston (not shown in Figures 2A-C) at the required distance to inject the dosage that was set, such that injection release button 208 is not required. For other pens, such as the Autopen 24, after setting the determined drug dosage 206, the drug is injected by releasing the injection release button 208 which releases the piston (not shown in Figures 2A-C) that pushes the syringe piston to the required distance to inject the desired dosage as set by dial 206. In some pens, for drug delivery, the drug is accomplished by setting the determined drug dosage 206 and pressing the needle release button 208, to release the needle (not shown in Figures 2A-C) into the targeted area and through needle housing 202, thereby injecting the required drug dosage through that needle. However, the drug delivery pen according to the present invention provides an additional treatment element, such as, in the form of another drug, to induce a treatment for improving the primary drug being injected. The above injection pen's configuration and mechanism as well as other injection devices, such as syringes or jet injectors, can be configured to be used with devices and methods (not claimed) for applying additional treatment to the vicinity of the drug injection site, as described by the present and related applications. In some embodiments, the primary drug can be insulin.

The treatment is delivered over the target tissue using the pen's treatment delivery openings 204, wherein a fluid treatment substance is applied onto the tissue. Figure 2C is a cross-sectional view of drug delivery pen 200 showing the treatment element 230 and components thereof. Treatment element 230 includes a treatment compartment 220, a plurality of valves 222, a pump 226, a treatment tube 224 and a treatment release button 228. The treatment compartment 220 stores a formulation, a fluid, such as a liquid, ointment, solution, aerosol, pressurized liquid, gas, spray, pain relieve drug, analgesic, vasodilatation drug, septic, alcohol, or the like for treating the skin tissue area. The treatment may be applied prior to or following deployment of the needle, in a two step process one end of the pen for substance treatment delivery using opening 204 and the opposite end for the injection using housing 202.

A treatment substance stored in the compartment 220 can be pumped to the treatment release openings 204 using schematic pump 226 that is activated by pressing the treatment release button 228. The treatment release button 228 releases valves 222 to deliver treatment liquid from the compartment 220 through the tube 224 that leads to the openings 204. In some implementations, the treatment applied to an area may lead to improved pharmacokinetic and/or pharmacodynamic properties of the primary drug being delivered by way of an injection. The pathway by which the pharmacodynamic and/or pharmacokinetic properties of the drug are improved may be dependent on the treatment substance or element utilized.

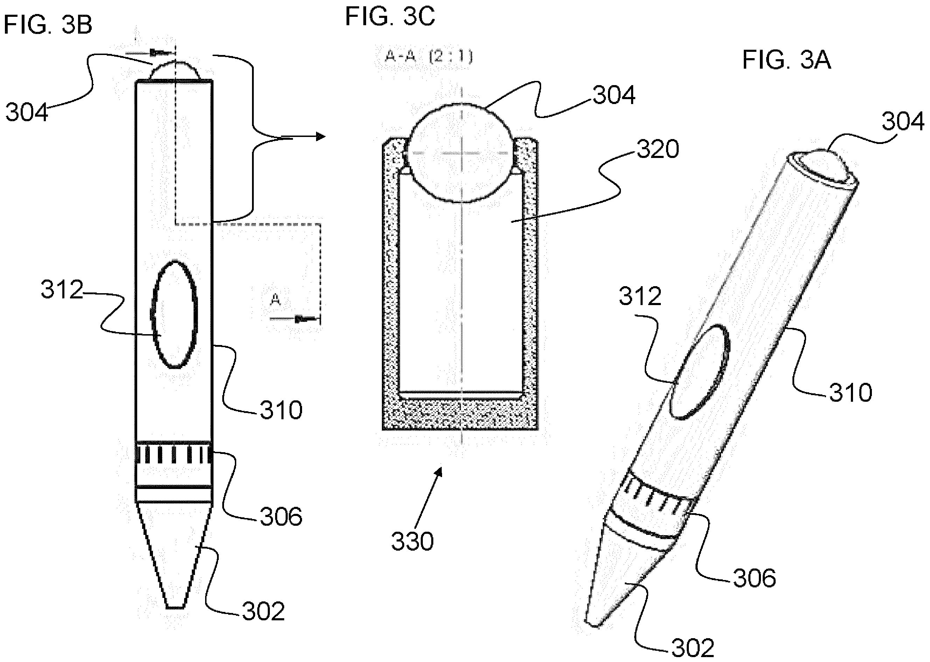

Figures 3A-C illustrate an exemplary pen-type drug delivery device similar to that described in connection with Figures 1A-E and 2A-C, however, in this case, the treatment substance is applied by way of a roller-applicator ball 304 onto the tissue being treated. Figures 3A and 3B illustrate similar views of drug delivery pen 300. The drug delivery function of pen 300 is similar to the functions of pens shown in Figures 1A-E and 2A-C. The drug dosage can be controlled and determined using a dosage dial 306 that is delivered via the needle housing 302 that encloses a needle (not shown in Figures 3A-C), which is triggered using an injection release button 312. A treatment substance may be applied to the injected area either prior to or following injection.

Figure 3C depicts treatment element 330 including treatment substance container 320 and substance application ball 304. The treatment compartment 320 stores a fluid, such as, a liquid, ointment, solution, aerosol, pressurized liquid, gas, spray, pain relieve drug, analgesic, vasodilatation drug, septic, alcohol, or the like for treating the skin tissue area. Treatment may be applied with a ball 304 prior to or following deployment of the needle, in at least a two-step process one end of the pen for substance treatment delivery using ball 304 the opposite end for the injection using housing 302.

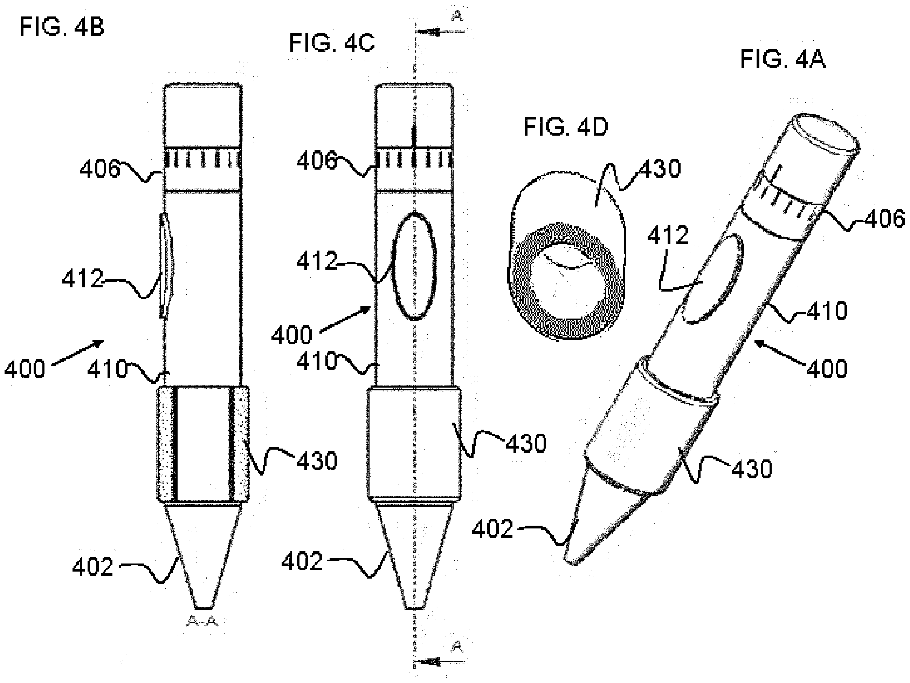

Figures 4A-C illustrate another exemplary drug delivery pen 400, according to some embodiments of the present invention. Treatment substance 430 used to improve the pharmacokinetic and/or pharmacodynamic properties of the drug being delivered, such as insulin, may come in the form of a solid, gel, gel form, thixotropic solution or the like. The substance 430 may be applied to a treatment area either prior to or following drug delivery using a needle delivered through needle housing 402. The treatment substance 430 can be a solid, stick, ointment, solution, pain relieving drug, analgesic, vasodilatation drug, septic, alcohol, or the like to treat the skin or tissue area. Substance treatment 430 can be rubbed, rolled over the treated area or applied in any other suitable way.

The drug delivery pen 400 may be manufactured separately from the individual treatment substance 430; alternative, the drug delivery pen 400 may be manufactured along with treatment substance 430 being coupled thereto. Figure 4D depicts the treatment substance 430 that can be configured to fit over the pen shaft 410 using a sticker, a threading, an adhesive layer, a clip, or any other coupling tools. The pen shaft 410 can be pushed through the lumen of treatment substance 430. The treatment substance 430 can be configured to fit circumferentially around the pen shaft 410. In some embodiments, a single use or a reusable treatment substance 430 can be used with other drug injection devices, such as a syringe.

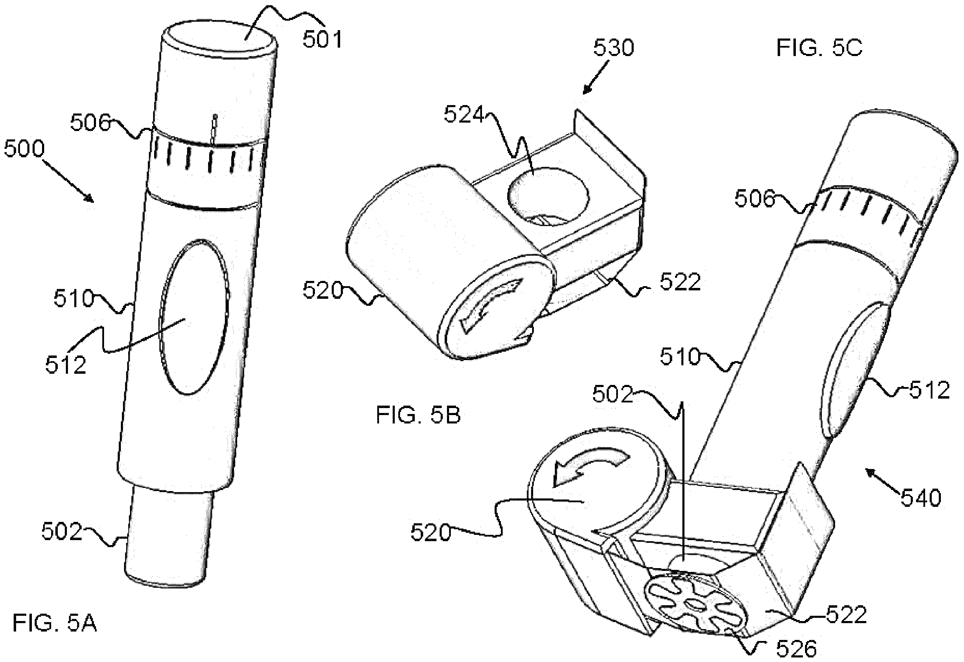

Figure 5A illustrates an exemplary drug delivery injectable pen having a needle housing that can accept secondary devices for substance treatment deployment or use, according to some embodiments of the present invention. Drug delivery pen 500 includes shaft 510 that is configured to contain a volume of the injectable drug (such as insulin) for delivery. A dosage dial 506 sets the dosage to be injected with a needle that is injected via an injection release button 520 through the needle and the needle housing 502.

In some embodiments, the drug delivery pen 500 includes a glucose sensor 501 for measuring blood glucose with a finger stick. Glucose sensor 501 can be configured to be on the top of the drug delivery pen 500. The pen 500 can be used to release a needle that is used to draw a drop of blood that may be placed over a finger stick (not shown in Figures 5A-C) and is placed over the glucose sensor 501 for reading the glucose levels in the drawn blood sample. Additionally, the drop of blood can be applied to a finger stick like glucose sensor inserted into a glucometer type slit, for example on the top of pen 500 (not shown in Figures 5A-C).

Figure 5B depicts a treatment element apparatus 530 that may be coupled to the drug delivery pen 500, as shown in Figure 5C, to form drug delivery apparatus 540. The drug delivery apparatus 540 including the treatment element apparatus 530 and drug delivery pen 510 may be single unit.

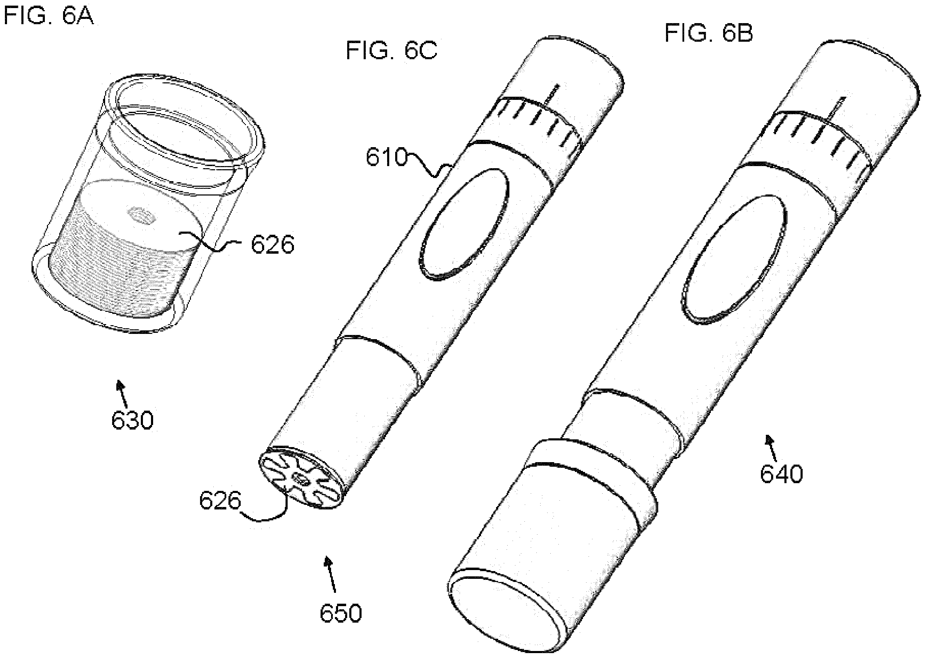

The treatment element apparatus 530 includes a female connector 524 that can be securely coupled to the needle housing 502, which has a corresponding male connector shape. The coupling can be undertaken by threading, fitting, pin lock assembly, adhesives or any other coupling methods. The drug delivery pen 530 includes treatment substance dispenser 520 that contains a roll 520 adhered to a plurality of treatment element 526. The treatment element 526 can be securely placed over the end of needle housing 502. The treatment element 526 can be implemented in the form of a pad (e.g., releasing an additional substance), a heating pad, a PCB heating element, an optical treatment element, an electromagnetic radiation treatment element, an electrical current treatment element, an acoustical treatment element, a massaging treatment element or an element related to any of the treatment methods discussed above. The treatment element 526 can deliver treatment to a target tissue prior to injection, following injection, or at the time of injection to improve the pharmacodynamic and/or pharmacokinetic properties of an injectable drug. Treatment element 526 can also include a power source to provide the desired treatment power. Treatment element can include a control element, such as an electrical circuit, to control the treatment profile. Treatment element can be disposable, e.g., single use treatment element, whereby after the treatment profile ends, the user can dispose of the treatment element 526. Treatment element can be reusable, whereby after the treatment profile ends, the user can recharge it, exchange the power source, or exchange only a portion of the treatment element 526 that is disposable and then reload it into a new or the same treatment substance dispenser 520 or a substance dispenser 630 shown in Figure 6A or any other configuration, as disclosed in the present application.

Figures 6A-C illustrate exemplary treatment element dispensers that may be coupled to a drug delivery pen 500, as illustrated in Figure 5A. Figure 6A illustrates a treatment dispenser 630 that is an optional alternative to dispenser 530 of Figure 5B. Treatment dispenser 630 includes stackable treatment elements 626 that may be coupled to the pen drug delivery device. Figure 6B illustrates how a drug delivery pen is coupled to the treatment element dispenser 630. Once the treatment element dispenser 630 is decoupled from the delivery pen apparatus 650, the needle end of the apparatus is coupled with a treatment element 626. The treatment element 626 is alignable with the needle of drug delivery pen 650. This alignment provides for a drug delivery and treatment apparatus 650 to induce application of treatment using element 626 prior to, following, or at the same time as undertaking drug delivery with the drug delivery pen 600 in a one step process.

In some embodiments, the drug delivery using the pen 600 and evoking treatment using element 626 may be undertaken in a two-step process, where treatment element 626 is coupled to a non-needle end of the drug delivery pen 600. This allows drug delivery and treatment to be performed individually. For example, one can trigger the drug delivery with the injection release button 612 and later evoke treatment using element 626. The reverse is also true where treatment may precede drug delivery.

In some embodiments, the treatment element, such as treatment element 526 (shown in Figures 5A-C) or element 626, is coupled to a single use needle that is secured to the pen before each dose injection (as shown in Figures 1D and IE), such that when the needle is inserted into the body, the treatment element is attached to and/or otherwise adheres to or around the injection site, automatically, without the need of additional operations by the user.

For any of the embodiments shown herein, the treatment element may include an energy source, which can provide heat, radiation, mechanical vibrations, suction, magnetic energy, ultrasound, light irradiation, RF irradiation, microwave irradiation, electrical stimulation, or any other form of energy or combinations of those energy sources. For example, the treatment element may include a heater to heat the injection site; or a source of optical energy for the energy source, such as a light source, including but not limited to LEDs or laser diodes for example, with one or more other optical elements; or a micro-wave generator or emitter configured to irradiate the injected region with micro-wave radiation; or a radio frequency electromagnetic radiation generator or emitter configured to irradiate the injected region with radio frequency electromagnetic radiation; or a vibration device configured to vibrate the injected region; or a vacuum device for applying suction to the injected region; or an electric field generator or emitter configured to apply an electric field to the injected region; or a magnetic field generator or emitter configured to apply magnetic field to the injected region; or an acoustic signal generator or emitter configured to apply acoustic stimulation to the injected region.

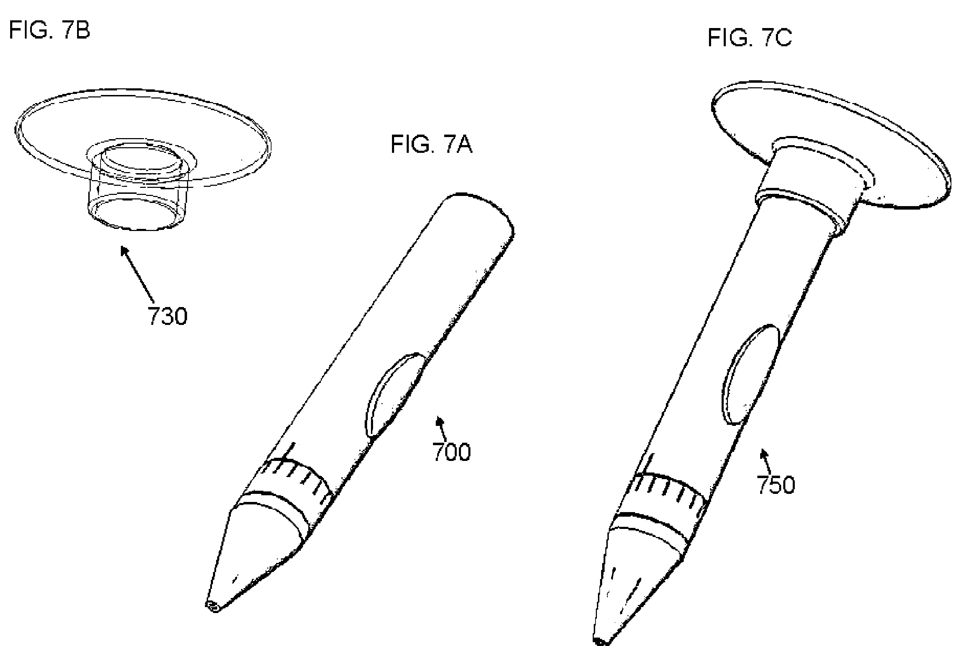

Figures 7A-C illustrates an exemplary drug delivery pen 700 similar to the pens shown in FIGS. 5A-C and 6A-C, that is coupled to a treatment element dispenser 730 as shown in Figure 7B to create drug delivery and treatment apparatus 750. The apparatus 750 includes a treatment element dispenser 730 at the non needle end of drug delivery pen 700. The apparatus 750 provides the drug delivery pen with an ability to deliver drug(s) in a chosen dosage while providing the treatment element that may improve the pharmacokinetic and/or pharmacodynamic property of the delivered drug. In some embodiments, the user can store his/her treatment element(s) in a special case, or in the case of the drug injection device. Before or after the drug injection, the user inserts the treatment element, as discussed above, into the treatment element dispenser 730 and applies it to the injection site. In some embodiments, the treatment element can have a similar shape as the treatment element dispenser 730 and can be coupled directly to the drug delivery pen 700 without using the treatment element dispenser as an adaptor.

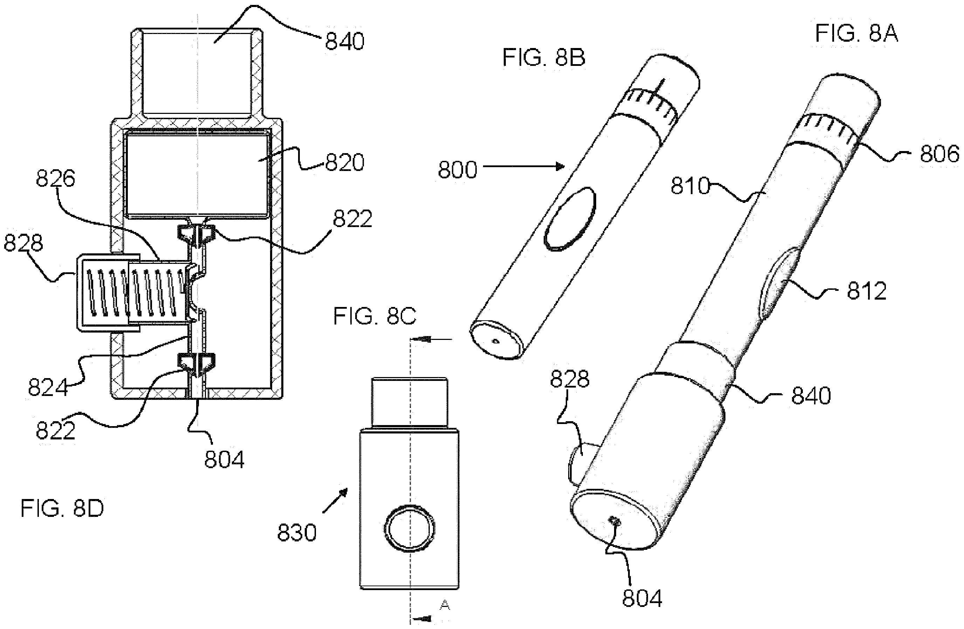

Figures 8A-D illustrate an exemplary treatment apparatus, similar to the one shown in FIGS. 1A-3C, according some embodiments of the present invention. Figure 8A depicts a drug delivery pen 800 used to deliver an injectable drug. Figures 8B and 8C are views of the treatment element 830 that can be coupled to the drug delivery pen 800, thus, forming a drug delivery and treatment apparatus, as shown in Figure 8A. The treatment element 830 can be coupled to the pen 800 via the opening 840 that receives the needle end of the drug delivery pen 800. Treatment element 830 includes a treatment substance compartment 820 that can be utilized to store treatment fluid. The treatment compartment 820 stores a fluid, such as gel, foam, liquid, ointment, solution, aerosol, pressurized liquid, gas, spray, pain relieve drug, analgesic, vasodilatation drug, septic, alcohol, or the like for treating the skin tissue area. Treatment can be applied prior to or following deployment of the needle.

Treatment liquid stored in the compartment 820 can be delivered to the target tissue through the opening 804 using pump 826 that is activated by pressing the treatment release button 828. The treatment release button 828 releases valves 822 to deliver treatment liquid from the compartment 820 through the tube 824 that leads to the opening 804. In some embodiments, the amount of treatment liquid can be preset for a user. In some embodiments, a special dial or other means, (not shown in Figures 8A-D, but illustrated in Figure IE), may be used to set the amount of applied treatment substance. The treatment applied to an area can lead to improved pharmacokinetic and/or pharmacodynamic properties of the primary drug being delivered by way of injection (e.g., insulin). The pathway by which the pharmacodynamic and/or pharmacokinetic properties of the drug are improved is optional and may be dependent on the treatment substance or element utilized.

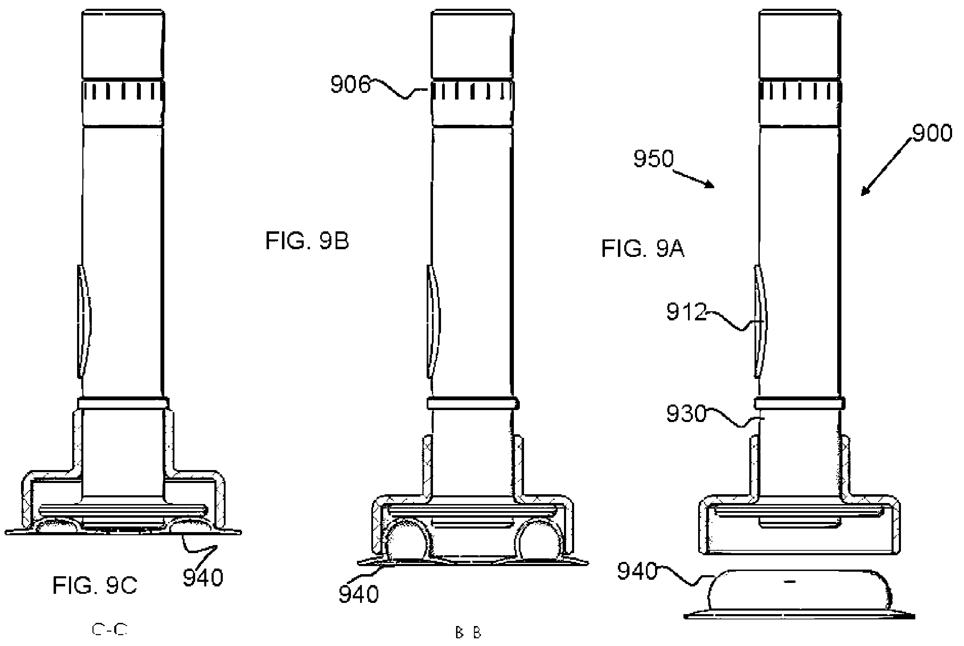

Figures 9A-C illustrate the drug delivery pen 900 having the treatment element provide pressure-related treatment, such as suction, massage, or the like, according to some embodiments of the present invention. Figure 9A depicts a drug delivery pen and treatment apparatus 950 including a drug delivery pen 900 and a treatment element attachment 930 that can be mechanically coupled. The apparatus 950 can be provided as an assembled or unitary drug delivery device. The apparatus 950 can be coupled to the treatment element 940 that is made from a pliable material that can withstand pressure. Such material can be rubber, latex, or any other suitable material configured to create suction over a given treatment area similar to a plunger. The apparatus 950 is placed over the treatment element 940 and is compressed to create further pressure, as shown in Figures 9B and 9C, or suction when the pen is lifted. Such suction will bring about vasodilatation in the treated tissue and is configured to improve pharmacodynamics and/or pharmacokinetics of the delivered drug. Drug delivery deployment can be undertaken in the compressed form of the treatment element 940, as shown in Figure 9C.

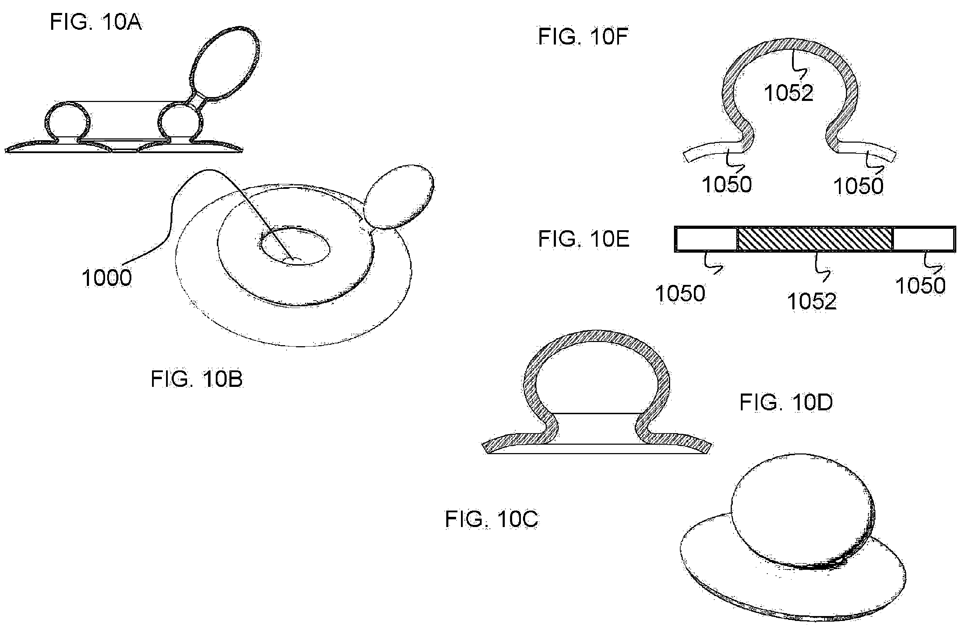

Figures 10A-D illustrate a variety of pressure based treatment elements, according to some embodiments of the present invention. Figure 10A is a cross-sectional view of the treatment element shown in Figure 10B. The treatment element can be used during drug delivery and includes a lumen 1000 allowing the needle to penetrate through. Figure 10C is a cross-sectional view of the treatment element shown in Figure 10D. The treatment element of Figure 10D can be utilized either prior to or following drug delivery in order to create suction over the tissue area. The treatment elements of Figures 10A-D can be used to create suction or vacuum in the vicinity of the drug injection site and to induce local vasodilatation.

Figures 10E and 10F illustrate an exemplary treatment element configured as a durable adhesive tape that is used to effectively pinch a fold of skin while maintaining its shape. The treatment element shown in Figure 10E includes two sticky ends 1050 that are bridged by a malleable section 1052. Each one of the ends 1050 is placed over a patch of skin where an injection for example with a syringe or a drug delivery pen is to be undertaken. Once in place, the ends 1050 are pushed toward each other by the malleable section 1052 to form a bell type shape. Section 1052 can be manufactured from a strong malleable material that can hold its shape, while being repeatedly formed and deformed. Figure 10F depicts the treatment element of Figure 10E in a folded form. Section 1052 can be moulded and reshaped forming a treatment element for example to bring about vasodilatation.

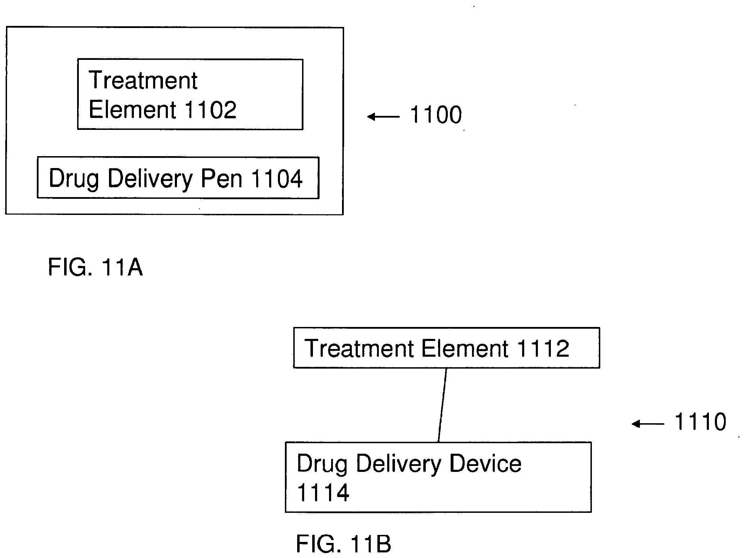

Figure 11A is a block diagram of an exemplary drug delivery apparatus 1100 having a treatment element 1102 incorporated into a drug delivery pen 1104, wherein the treatment element is integrated into the drug delivery pen, according to some embodiments of the present invention. Figure 11B is a block diagram of an exemplary drug delivery apparatus 1110 having a treatment element 1112 and a drug delivery device 1114 that are removably coupled to each other, according to some embodiments of the present invention. The drug delivery device 1114 or treatment element 1112 can function independently of one another and can be securely coupled to each other to form a single drug delivery and treatment apparatus 1110, similar to the embodiment of Figures 4A-6C. Drug delivery device 1114 can be implemented as a syringe, drug delivery pen, drug delivery jet injector or the like.

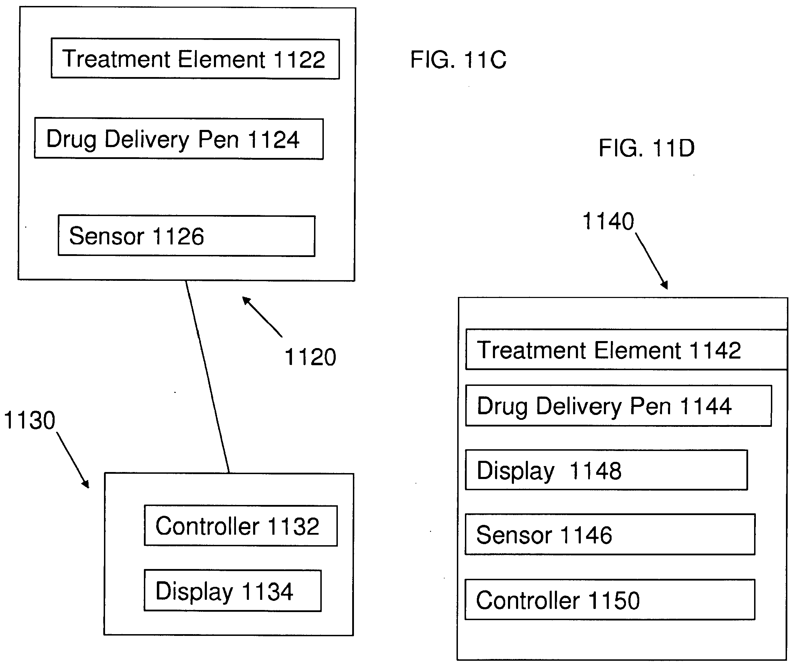

Figure 11C is a block diagram of an exemplary drug delivery and treatment apparatus that further includes a sensor 1126, such as a glucose stick sensor, for measuring blood glucose. The drug delivery apparatus 1120 communicates with an external processing unit 1130. The processing unit can be a PDA, a cellular phone, a computer, a laptop or any other device. The unit 1130 includes a controller 1132 and a display 1134. The controller 1132 controls analysis of data received from the drug delivery apparatus 1120 to determine treatment or dosage form or the like related to the functioning of drug delivery pen 1124, and treatment element 1122. The processing unit 1130 provides the user with data regarding historical and current use of the drug delivery apparatus 1120.

Figure 11D is a block diagram of an exemplary drug delivery device similar to the device shown in Figure 11C, where the drug delivery apparatus 1140 has an integrated sensor 1146, a processing unit 1150 and a display 1148, according to some embodiments of the present invention. This allows the user to fully control, visualize all activity related to the drug delivery pen 1144 or the treatment element 1142.

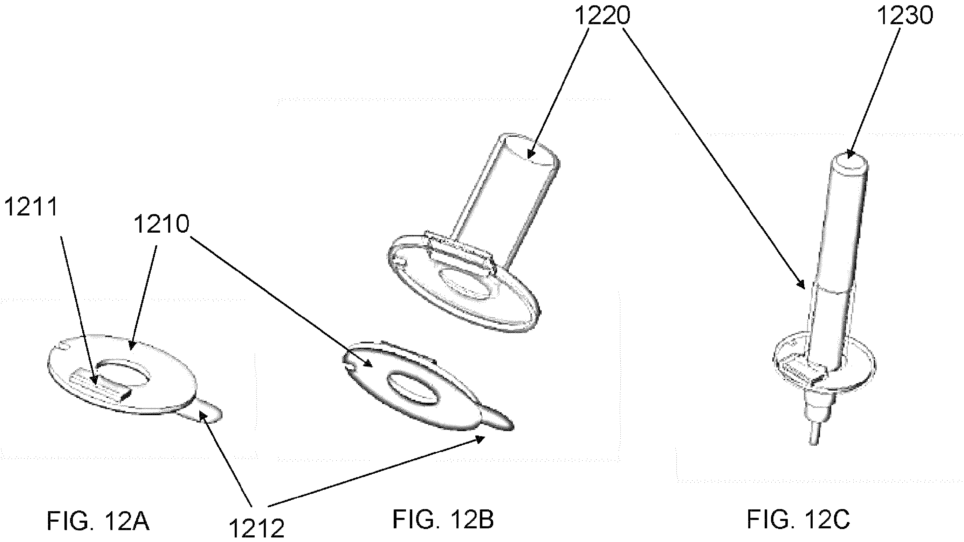

Figures 12A-C schematically illustrate exemplary treatment element dispensers that may be coupled to a drug injection device 1200 (as shown in Figure 12C), according to some embodiments of the present invention. Figure 12A illustrates a treatment element 1210 with a power source 1211. In some embodiments, the treatment element 1210 includes a power source and a control element to control a treatment profile. For instance, in case of heating, treatment element 1210 can include a heater to heat the tissue around the injection site to a temperature that improves drug's pharmacokinetics and pharmacodynamics. In case of temperature sensitive drugs, such as insulin, treatment element 1210 can include a heater to heat the tissue around the injection site to a temperature that improves drug's pharmacokinetics and pharmacodynamics, without heating the drug above a limiting temperature that may degrade it, such as 37°C in the case of some types of insulin. Treatment element 1210 may include also an adhesive layer on its bottom side covered with a laminate 1212.

In some embodiments the user has a case with one or few treatment elements 1210. When the user wants to use treatment element over drug injection site, the user takes adaptor 1220, which can be stored in the same case or a different one, and attaches one treatment element 1210 to adaptor 1220, as shown in Figure 12B. Treatment element 1210 can be attached to adaptor 1220 with a weak mechanical locking, such as a plastic clip, or weak adhesive or other ways known in the art. Then laminate 1212 can be removed. Afterwards, adaptor 1220 is assembled or threaded over drug delivery pen or syringe 1230, as shown at Figure 12C. In some embodiments needle cap 1231, can be removed from the needle. Then, the needle is inserted into injection site tissue, the drug injection device is operated and the drug is injected through needle into the tissue. During that time, or slightly before or after adaptor 1230, is pushed down to the tissue and treatment element 1210 is attached to the tissue around drug injection site.

In some embodiments, the attachment of treatment element 1210 to the tissue is configured to activate it automatically and the treatment starts according to a predetermined treatment profile. This function can be performed, for example, by a small switch which is pressed when treatment element 1210 is attached to the tissue. In some embodiments, the treatment element 1210 can be activated manually. In some embodiments, treatment element can be controlled and/or programmed for a specific treatment element through a remote control or a connection to its case. Afterwards, the injection device 1230 and the adaptor 1220 are lifted off, either together or separately, and the treatment element 1210 is left attached to the tissue and applies treatment to the vicinity of the drug injection site. The user can remove treatment element after treatment ends or later on. In some embodiments, the treatment element includes an indicator for the user that indicates the beginning of the treatment and the end of the treatment. In some embodiments, the treatment element 1210 may be disposable.

In some embodiments, the treatment element's 1210 power source 1211 may be rechargeable, so that after the treatment ends and the user removes it from the skin, it can be put back to the case and/or placed in a charging cradle for recharging which may be disposed in said case. In some embodiments, the treatment element 1210 may have a disposable portion and a reusable portion. In some embodiments, the drug injection device and at least one treatment element are disposed in the same case prior to injection. This provides additional comfort for the user and allows the user to use both of them together or one after the other.

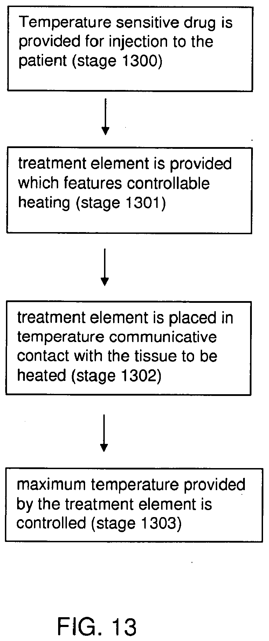

Figure 13 is a flow chart depicting a method for controlling the temperature of heating provided by a treatment elements that heat the injection site tissue vicinity in order to prevent degradation of a temperature sensitive drug. As shown in step 1300, a drug is provided for injection to the patient, where the drug is sensitive to degradation above a limiting temperature. In step 1301, a treatment element is provided that features a controllable heating through a controllable heating element. In step 1302, the treatment element is placed in thermal contact with the tissue to be heated, such that heat from the treatment element is transferred to the tissue to be heated.

In step 1303, a maximum temperature provided by the treatment element is controlled, such that the temperature experienced by the drug (that is, in the environment of the drug) does not exceed the limiting temperature sustainable by the drug before degradation occurs. In some embodiments, he maximum temperature can be calibrated for each drug and/or class of drugs. For example, for some types of insulin, the limiting temperature is about 37° C.

In some embodiments, such control can be provided through a microprocessor or other processor for controlling the temperature output by a heating element. A sensor can be provided in order to measure the temperature at and/or near the tissue being heated, in order to determine the temperature experienced by the drug.

The treatment element includes one or more materials capable of generating an exothermic reaction, in which the amount of such materials and/or ratio can be calculated in order for the temperature of the reaction not to exceed the maximum temperature set for the treatment element based on the desired limiting temperature of the drug. The exothermic reaction can be a heat-generating oxidation reaction, for example, with a mixture of iron powder, activated carbon, salt and water. As can be understood by one skilled in the art, other such mixtures of materials can be used.

In some embodiments, the treatment element and the drug delivery pen are not disposed in the same housing. However, in such cases the user may forget to apply the treatment to the injection site in some cases, thereby changing the pharmacokinetics, which is undesirable. Therefore, to prevent the user forgetting to apply the treatment to the injection site, the drug delivery pen can include a mechanism for reminding the user to apply the required treatment, before, during or after injecting the drug
into the tissue. In some embodiments, the drug delivery pen includes a mechanism that identifies whether the treatment was applied or not and permits drug injection only when the tissue treatment was applied. In some embodiments, the drug delivery pen includes, in addition to the drug injection mechanism, a sensor that indicates whether the treatment was applied or was not applied and a processing unit that enables injection of the drug only when the tissue treatment was applied. Such sensor can be an optical sensor that measures optical properties of the local tissue, or Laser Doppler Flowmeter ("LDF") that can measure local blood perfusion and identify that the vasodilatation inducing local treatment was applied and that the treatment level was adequate.

Example embodiments of the components of the present invention and exemplary methods have been described herein. As noted elsewhere, these example embodiments have been described for illustrative purposes only, and are not limiting. Other embodiments are possible and are covered by the invention. For example, at the present application many of the suggested methods (not claimed) and devices can be used for many of the drug injection devices, such as injection pens or syringes or jet injector and other known in the art injection devices, so although the examples are mainly given for injection pens they are applied to the other injection devices as well. Such embodiments will be apparent to persons skilled in the relevant art(s) based on the teachings contained herein. Thus, the scope of the present invention should not be limited by any of the above described exemplary embodiments, but should be defined only in accordance with the following claims and their equivalent.

## Claims

1. A drug delivery device (100) comprising:
a drug delivery housing (160) comprising a drug reservoir for containing the drug;
a piston for injecting the drug from the drug reservoir through an injection site via a needle housing (102);
**characterized by**:
a treatment element (130) comprising a treatment compartment (120);
a treatment substance for improving the pharmacokinetic and/or pharmacodynamic properties of the drug and stored in the compartment (120),
the treatment element being coupled to the drug delivery housing, wherein the needle housing comprises a needle opening and the treatment substance is delivered to the injection site through at least one of:
treatment delivery openings (104) formed in the needle housing (102) and surrounding the needle opening;
an opening (204) formed at an opposite end of the device to the needle housing (102); and
the needle opening.

2. The device according to claim 1, wherein the drug delivery device is one of a syringe, an injection pen, or a jet injector.

3. The device according to claim 1, wherein the compartment is an integral part of the drug delivery housing.

4. The device according to claim 1, wherein the compartment is configured to be removable from the housing and further configured to be interchanged, replaced or added to the drug delivery housing.

5. The device according to claim 1, wherein the drug is insulin.

6. The device according to claim 1, further comprising:
a drug dosage selector for selecting the required dosage of the drug; and
a treatment dosage selector for determining a dosage of the treatment substance to be applied.

7. The device according to any one of claims 1-6, wherein the drug delivery device includes a nozzle for jet-injecting the drug from the drug reservoir through the nozzle with a sufficient velocity to penetrate tissue of the patient to the injection site, wherein an energy source associated with the drug reservoir forces the drug through the nozzle with sufficient velocity to penetrate to the tissue of the patient to the injection site.

8. The device according to claim 7, wherein the energy source includes at least one of the following: a spring, a coil spring, and a gas spring, generally further comprising a trigger mechanism for activating the energy source for forcing the drug through the nozzle.

9. The device according to any claim 1-8, further comprising a sensor for indicating whether the treatment substance was applied, wherein a processing unit enables injection of the drug only when the tissue treatment was applied.

10. The device according to claim 9, wherein the sensor includes at least one of the following: an optical sensor for measuring optical properties of a local tissue of a patient, and Laser Doppler Flowmeter for measuring local blood perfusion and identifying that the vasodilatation inducing local treatment was applied and that the treatment level was adequate.

11. The device according to claim 5, further comprising a glucose sensor (1126) for detecting and measuring a level of glucose in the body of a patient and further comprising a controller (1132),wherein the controller is configured to apply a dosage of the treatment substance based on the measured level of glucose in the body of the patient.

12. The device according to claim 1 further comprising a treatment release button (128) that delivers the treatment substance to a targeted area via openings (104).

## Patentansprüche

1. Arzneimittelabgabevorrichtung (100), die Folgendes umfasst:
ein Arzneimittelabgabegehäuse (160), das ein Arzneimittelreservoir zur Aufnahme des Arzneimittels umfasst;
einen Kolben zum Injizieren des Arzneimittels aus dem Arzneimittelreservoir durch eine Injektionsstelle über ein Nadelgehäuse (102);
**gekennzeichnet durch**:
ein Behandlungselement (130), das eine Behandlungskammer (120) umfasst;
eine Behandlungssubstanz zur Verbesserung der pharmakokinetischen und/oder pharmakodynamischen Eigenschaften des Arzneimittels, die in der Kammer (120) gelagert ist,
wobei das Behandlungselement mit dem Arzneimittelabgabegehäuse verbunden ist, wobei das Nadelgehäuse eine Nadelöffnung umfasst und die Behandlungssubstanz über mindestens eines von Folgendem an die Injektionsstelle abgegeben wird:
Behandlungsabgabeöffnungen (104), die in dem Nadelgehäuse (102) gebildet sind und die Nadelöffnung umgeben;
eine Öffnung (204), die an einem gegenüberliegenden Ende der Vorrichtung zu dem Nadelgehäuse (102) gebildet ist; und
die Nadelöffnung.

2. Vorrichtung nach Anspruch 1, wobei die Arzneimittelabgabevorrichtung eine Spritze, ein Injektionsstift oder ein Strahlinjektor ist.

3. Vorrichtung nach Anspruch 1, wobei die Kammer ein einstückiger Teil des Arzneimittelabgabegehäuses ist.

4. Vorrichtung nach Anspruch 1, wobei die Kammer dafür konfiguriert ist, aus dem Gehäuse entfernbar zu sein, und ferner dafür konfiguriert ist, ausgetauscht, ersetzt oder dem Arzneimittelabgabegehäuse hinzugefügt zu werden.

5. Vorrichtung nach Anspruch 1, wobei das Arzneimittel Insulin ist.

6. Vorrichtung nach Anspruch 1, die ferner Folgendes umfasst:
einen Arzneimitteldosisselektor zum Auswählen der erforderlichen Dosis des Arzneimittels; und
einen Behandlungsdosisselektor zum Bestimmen einer Dosierung der zu applizierenden Behandlungssubstanz.

7. Vorrichtung nach einem der Ansprüche 1 bis 6, wobei die Arzneimittelabgabevorrichtung eine Düse umfasst, um das Arzneimittel aus dem Arzneimittelreservoir durch die Düse mit einer ausreichenden Geschwindigkeit per Strahl zu injizieren, um das Gewebe des Patienten bis zur Injektionsstelle zu durchdringen, wobei eine Energiequelle, die dem Arzneimittelreservoir zugeordnet ist, das Arzneimittel durch die Düse mit ausreichender Geschwindigkeit drückt, um in das Gewebe des Patienten zur Injektionsstelle einzudringen.

8. Vorrichtung nach Anspruch 7, wobei die Energiequelle mindestens eines der Folgenden umfasst: eine Feder, eine Schraubenfeder und eine Gasfeder und im Allgemeinen ferner einen Auslösemechanismus zum Aktivieren der Energiequelle umfasst, um das Arzneimittel durch die Düse zu drücken.

9. Vorrichtung nach einem der Ansprüche 1 bis 8, die ferner einen Sensor umfasst, der anzeigt, ob die Behandlungssubstanz aufgetragen wurde, wobei eine Verarbeitungseinheit die Injektion des Arzneimittels nur dann ermöglicht, wenn die Gewebebehandlung angewendet wurde.

10. Vorrichtung nach Anspruch 9, wobei der Sensor mindestens eines von Folgendem umfasst: einen optischen Sensor zum Messen optischer Eigenschaften eines lokalen Gewebes eines Patienten und einen Laser-Doppler-Durchflussmesser zur Messung der lokalen Durchblutung und zur Feststellung, dass die Vasodilatation induzierende lokale Behandlung angewendet wurde und dass das Behandlungsniveau angemessen war.

11. Vorrichtung nach Anspruch 5, die ferner einen Glucosesensor (1126) zum Erfassen und Messen eines Glukosespiegels im Körper eines Patienten umfasst und die ferner eine Steuereinheit (1132) umfasst, wobei die Steuereinheit dafür konfiguriert ist, eine Dosierung der Behandlungssubstanz basierend auf dem gemessenen Glukosespiegel im Körper des Patienten anzuwenden.

12. Vorrichtung nach Anspruch 1, die ferner einen Behandlungsfreigabeknopf (128) umfasst, der die Behandlungssubstanz über Öffnungen (104) an einen Zielbereich abgibt.

## Revendications

1. Dispositif d'administration de médicaments (100) comprenant :
un logement d'administration de médicament (160) comprenant un réservoir de médicament destiné à contenir le médicament ;
un piston destiné à injecter le médicament depuis le réservoir de médicament à travers un site d'injection via un logement d'aiguille (102) ;
**caractérisé par** :
un élément de traitement (130) comprenant un compartiment de traitement (120) ;
une substance thérapeutique destinée à améliorer les propriétés pharmacocinétiques et/ou pharmacodynamiques du médicament et stockée dans le compartiment (120),
l'élément de traitement étant couplé au logement d'administration de médicament, dans lequel le logement d'aiguille comprend une ouverture pour l'aiguille et la substance thérapeutique est acheminée vers le site d'injection à travers au moins l'une des ouvertures ci-dessous :
des ouvertures d'administration de traitement (104) formées dans le logement d'aiguille (102) et entourant l'ouverture pour l'aiguille ;
une ouverture (204) formée à une extrémité opposée du dispositif par rapport au logement d'aiguille (102) ; et
l'ouverture pour l'aiguille.

2. Dispositif selon la revendication 1, dans lequel le dispositif d'administration de médicaments est soit une seringue, soit un stylo d'injection, soit un injecteur à jet.

3. Dispositif selon la revendication 1, dans lequel le compartiment fait partie intégrante du logement d'administration de médicament.

4. Dispositif selon la revendication 1, dans lequel le compartiment est configuré pour être amovible du logement et est en outre configuré pour être changé, remplacé ou ajouté au logement d'administration de médicament.

5. Dispositif selon la revendication 1, dans lequel le médicament est de l'insuline.

6. Dispositif selon la revendication 1, comprenant en outre :
un sélecteur de dose de médicament destiné à sélectionner la dose requise du médicament ; et
un sélecteur de dose de traitement destiné à déterminer une dose de la substance thérapeutique à appliquer.

7. Dispositif selon l'une quelconque des revendications 1 à 6, dans lequel le dispositif d'administration de médicaments comprend une buse destinée à injecter par jet le médicament depuis le réservoir de médicament à travers la buse, à une vélocité suffisante pour qu'il pénètre le tissu du patient jusqu'au site d'injection, dans lequel une source d'énergie associée au réservoir de médicament expulse le médicament à travers la buse, à une vélocité suffisante pour qu'il pénètre le tissu du patient jusqu'au site d'injection.

8. Dispositif selon la revendication 7, dans lequel la source d'énergie comprend au moins l'une des sources suivantes : un ressort, un ressort hélicoïdal, et un ressort pneumatique, comprenant généralement en outre un mécanisme de déclenchement destiné à activer la source d'énergie pour expulser le médicament à travers la buse.

9. Dispositif selon l'une quelconque des revendications 1 à 8, comprenant en outre un capteur destiné à indiquer si la substance thérapeutique a été appliquée, dans lequel une unité de traitement permet l'injection du médicament uniquement lorsque le traitement du tissu a été appliqué.

10. Dispositif selon la revendication 9, dans lequel le capteur comprend au moins l'un des éléments suivants : un capteur optique destiné à mesurer les propriétés optiques d'un tissu local d'un patient, et un débitmètre à technologie laser et Doppler destiné à mesurer le débit sanguin local et à identifier que le traitement local induisant la vasodilatation a été appliqué et que le niveau de traitement est adéquat.

11. Dispositif selon la revendication 5, comprenant en outre un capteur de glucose (1126) destiné à détecter et à mesurer un taux de glucose dans l'organisme d'un patient et comprenant en outre un dispositif de commande (1132), dans lequel le dispositif de commande est configuré pour appliquer une dose de la substance thérapeutique en fonction du taux de glucose mesuré dans l'organisme du patient.

12. Dispositif selon la revendication 1, comprenant en outre un bouton d'administration de traitement (128) qui délivre la substance thérapeutique vers une zone cible via des ouvertures (104).
